(19) Europäisches Patentamt
European Patent Office
Office européen des brevets

(11) EP 2 490 655 B1

(12) EUROPÄISCHE PATENTSCHRIFT

(45) Veröffentlichungstag und Bekanntmachung des Hinweises auf die Patenterteilung:
**04.01.2017 Patentblatt 2017/01**

(21) Anmeldenummer: **10766296.7**

(22) Anmeldetag: **21.10.2010**

(51) Int Cl.:
***A61K 8/73*** (2006.01) ***A61Q 5/06*** (2006.01)
***A61K 8/04*** (2006.01) ***A61K 8/06*** (2006.01)

(86) Internationale Anmeldenummer:
**PCT/EP2010/065863**

(87) Internationale Veröffentlichungsnummer:
**WO 2011/048179 (28.04.2011 Gazette 2011/17)**

# (54) ZUSAMMENSETZUNGEN ZUR FORMGEBUNG KERATINISCHER FASERN ENTHALTEND MIT PROPYLENOXID MODIFIZIERTE STÄRKE

COMPOSITION FOR SHAPING KERATIN FIBRES CONTAINING STARCHES MODIFIED WITH PROPYLENE OXIDE

COMPOSITION POUR LA MISE EN FORME DES FIBRES KÉRATINIQUES, CONTENANT UN AMIDON MODIFIÉ PAR DE L’OXYDE DE PROPYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2009 DE 102009045933**

(43) Veröffentlichungstag der Anmeldung:
**29.08.2012 Patentblatt 2012/35**

(73) Patentinhaber: **Henkel AG & Co. KGaA**
**40589 Düsseldorf (DE)**

(72) Erfinder:
- **SCHWEINSBERG, Matthias**
  **22769 Hamburg (DE)**
- **KNAPPE, Thorsten**
  **22869 Schenefeld (DE)**
- **RÖNISCH, Ralf**
  **42349 Wuppertal (DE)**
- **SCHRIEFERS, Mathias**
  **41239 Mönchengladbach (DE)**
- **DOGAN, Carine**
  **F-91270 Vigneux sur Seine (FR)**

(56) Entgegenhaltungen:
**EP-A1- 0 659 394** **EP-A2- 0 948 958**
**EP-A2- 0 948 959** **EP-A2- 0 948 960**
**EP-A2- 1 317 916**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische Mittel zur temporären Umformung keratinischer Fasern, enthaltend in einem in Form eines dispersen Systems vorliegenden, kosmetischen Träger mindestens eine mit Propylenoxid modifizierte Stärke, welche ein mittleres Molekulargewicht (Gewichtsmittel) von 700 bis 1000 kDa besitzt.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Die in Mitteln zur temporären Formgebung üblicherweise eingesetzten synthetischen Polymere werden aus entsprechenden synthetisch zugänglichen Monomeren hergestellt. Besagte Monomere werden aus fossilen Stoffen wie beispielsweise Erdöl durch Umwandlung zu den entsprechenden Polymerbausteinen u.a. unter Aufwand von Energie gewonnen.

Im Rahmen eines nachhaltigeren Umganges mit der Natur als Lebensraum und mit den Resourcen bleibt es wünschenswert, für kosmetische Produkte nur solche kosmetische Rohstoffe zu verwenden, die unter möglichst wenig Einsatz von Energie aus so genannten nachwachsenden Rohstoffen zugänglich sind. Allerdings kann eine Mengenreduktion oder gar ein Austausch besagter synthetischer Polymere nur dann vorgenommen werden, wenn der Ersatz die für den Anwendungszweck gewünschten Eigenschaften aufweist und den keratinhaltigen Fasern einen ausreichenden, stabilen Halt Form gewährleist.

Des weiteren sollen die Ersatzpolymere auf natürlicher Basis die Sprungkraft und Geschmeidigkeit der in der Form fixierten, keratinhaltigen Fasern erhalten. Die Bildung von mit dem bloßen Auge sichtbaren Polymerpartikeln auf den keratinhaltigen Fasern muss vermieden werden. Ferner darf die keratinhaltige Faser nicht stumpf wirken, sondern soll natürlich glänzen. Aufgabe der vorliegenden Erfindung war es daher, eine formfixierend wirkende, kosmetische Zusammensetzung bereitzustellen, wobei zur Formfixierung auf vorwiegend nachwachsende Rohstoffen zurückgriffen werden soll. Die Zusammensetzung soll ferner eine verbesserte Formfixierung bewirken und die vorgenannten Nachteile nicht aufweisen.

Ein erster Gegenstand der Erfindung ist daher ein kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke, welche ein mittleres Molekulargewicht (Gewichtsmittel) von 700 bis 1000 kDa besitzt. Diese Mittel weisen hervorragende Parameter in der Anwendung am Haar auf. Die besagte Stärke lässt sich nahezu ohne Einsatz von Wärme bei höchstens 30°C durch bloßes Mischen in die erfindungsgemäßen Mittel einarbeiten.

Unter keratinischen Fasern sind dabei erfindungsgemäß Pelze, Wolle, Federn und insbesondere menschliche Haare zu verstehen.

Der kosmetische Träger liegt für sich genommen, d.h. ohne besagte mit Propylenoxid modifizierte Stärke, zwingend als disperses System vor. Unter einem dispersen System ist ein System aus mehreren Phasen zu verstehen, wobei in einer kontinuierlichen, flüssigen Phase eine weitere Phase in Form eines Feststoffs und/oder einer Flüssigkeit und/oder eines Gases fein und nahezu gleichmäßig über die kontinuierliche Phase verteilt vorliegt. Insbesondere liegt der kosmetische Träger in Cremeform oder Gelform oder Schaumform vor.

Unter einer Creme ist erfindungsgemäß eine Zusammensetzung in Form eines dispersen Systems zu verstehen, die neben Wasser zusätzlich Fett und/oder Öl enthält. Bevorzugt geeignete Fette bzw. Öle werden im Verlaufe dieser Anmeldung beschrieben (*vide infra*).

Unter einem Gel ist erfindungsgemäß ein formbeständiges, leicht deformierbares und disperses System zu verstehen, das aus einem kolloid zerteilten Stoff mit langen oder stark verzweigten Teilchen und einer Flüssigkeit als Dispersionsmittel bestehen. Als lange oder stark verzweigte Teilchen dienen bevorzugt polymere Verdickungsmittel (*vide infra*).

Unter einem Schaum ist erfindungsgemäß ein disperses System zu verstehen, in dem ein Gas als zerteilte Phase in einer Flüssigkeit als kontinuierliche Phase vorliegt.

Die erfindungsgemäßen Mittel enthalten ihre Wirkstoffe in einem in Form eines dispersen Systems vorliegenden, kosmetischen Träger, bevorzugt in

- einem wasserhaltigen, in Form eines dispersen Systems vorliegenden kosmetischen Träger,

- einem alkoholischen in Form eines dispersen Systems vorliegenden, kosmetischen Träger oder
- einem wässrig-alkoholischen, in Form eines dispersen Systems vorliegenden kosmetischen Träger.

Das disperse System wird für diese Träger wie oben geschildert bevorzugt ausgewählt unter Creme, Gel, Schaum.

Unter wässrig-alkoholischen Trägern sind im Sinne der vorliegenden Erfindung wässrige Zusammensetzungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol, 1,2-Propylenglykol oder 1,3-Propylenglykol enthalten. Bevorzugt sind dabei alle wasserlöslichen organischen Lösemittel.

Das erfindungsgemäße Mittel enthält in besagtem kosmetischen Träger ein spezielles Stärkederivat.

Stärke ist ein Reservekohlenhydrat, das von vielen Pflanzen in Form von üblicherweise 1 bis 200 $\mu$m großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare mit Propylenoxid modifizierte Stärke kann sich aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch mit Propylenoxid modifizierte Tapioka Stärke bzw. mit Propylenoxid modifizierte Kartoffelstärke bzw. durch Gemische beider vorgenannter Stärken, erzielt. Ganz besonders bevorzugt enthält das erfindungsgemäße Mittel mindestens eine mit Propylenoxid modifizierte Kartoffelstärke.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die mit Propylenoxid modifizierte Stärke einen Amylosegehalt von kleiner 25 Gew.-%, insbesondere von kleiner 20 Gew.-%, -jeweils bezogen auf das Gewicht der besagten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear $\alpha$-1,4-glycosidisch verknüpfter d-Glucose, $M_r$ 50000-150000. Die resultierenden Ketten bilden in der Stärke Doppelhelices.

Amylopektin enthält neben den für Amylose beschriebenen $\alpha$-1,4-Verknüpfungen auch in einer Menge von 4 bis 6% $\alpha$-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer mit Propylenoxid modifizierten Stärke wird erfindungsgemäß ein Reaktionsprodukt aus einer Stärke und Propylenoxid verstanden. Ein solches Reaktionsprodukt umfasst mindestens eine Struktureinheit der Formel (PS),

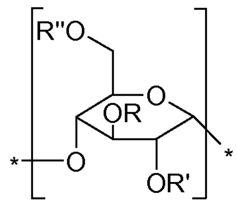 (PS),

worin mindestens ein Rest R, R' oder R" für eine Gruppe der Formel

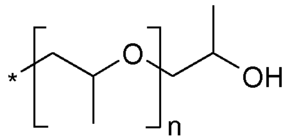

mit n $\geq 0$ steht
und maximal 2 der Reste aus R, R', R" für ein Wasserstoffatom steht. Die mit Propylenoxid modifizierten Stärken werden beispielsweise durch Umsetzung einer nativen Stärke mit Propylenoxid bereitgestellt. Vor der Modifikation mit Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung, etc.

Es ist erfindungsgemäß bevorzugt, wenn die mit Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mittels Propylenoxid modifiziert.

Die erfindungsgemäßen Propylenoxid modifizierten Stärken liegen in dem erfindungsgemäßen Mittel in einer Molekulargewichtsverteilung vor. Die Molekulargewichtsverteilung wurde experimentell mittels Gelfiltrationschromatographie gegen Dextran bestimmt. Ein wichtiges Merkmal der Erfindung ist das Gewichtsmittel des mittleren Molekulargewichts der in dem erfindungsgemäßen Mittel enthaltenen Propylenoxid modifizierten Stärken. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus *i* Segmenten (z. B. Monomerbausteinen) der Masse $M_i$ bestehen und in der Probe $N_i$-mal vorkommen. Für das Gewichtsmittel des Molekulargewichts $M_w = \Sigma w_i M_i$ gilt damit

$$M_w = [\Sigma(N_i M^2_i)] / [\Sigma(N_i M_i)].$$

Erfindungsgemässe Mittel enthalten solche mit Propylenoxid modifizierten Stärken, die ein mittleres Molekulargewicht (Gewichtsmittel) von 700 bis 1000 kDa, aufweisen. Die erfindungsgemäßen Mittel dieser Ausführungsform sind bevorzugt in Schäume oder Gele als kosmetischer Träger eingearbeitet und liegen daher als Schaum oder Gel vor. Die erfindungsgemäßen Mittel dieser Ausführungsform sind bevorzugt in Schäume oder Gele als erfindungsgemäßen Mittel dieser Ausführungsform sind bevorzugt in Schäume oder Gele als kosmetischer Träger eingearbeitet und liegen daher als Schaum oder Gel vor. Trotz des für festigende Polymere geringen Molekulargewichts lässt sich mit diesen besagten Stärken eine hervorragende Festigung erzielen. Ferner erreichen die mit besagten Stärken hergestellten Gele zusätzlich eine superbe Transparenz.

Zur Einstellung des Molekulargewichts wird die Stärke vor oder nach der Modifizierung mit Propylenoxid einer mechanischen und/oder einer chemischen Behandlung unterzogen. Zur Molekulargewichtserhöhung kann die besagte Stärke vernetzt werden. Eine Vernetzung der mit Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln durchführen. (Mit Propylenoxid modifizierte) Stärken, die eine solche Vernetzung nicht aufweisen, sind unvernetzt.

Eine Vernetzung erfolgt beispielsweise mit Epichlorhydrin. Hierzu wird eine 42 Gew.-%-ige Mischung von mit Propylenoxid modifizierter Stärke in Wasser hergestellt, in diese die gewünschte Menge Epichlorhydrin bei Raumtemperatur eingerührt und nach einer Rührzeit von 1 bis 5 Stunden unter Kontrolle der Viskosität nach erreichen der Zielviskosität die vernetzte Stärke nach gängigen Verfahren isoliert.

Es ist jedoch erfindungsgemäß besonders bevorzugt, wenn die erfindungsgemäßen Mittel mindestens eine unvernetzte, mit Propylenoxid modifizierte Stärke enthalten.

Zur Erzielung des niedrigeren Molekulargewichts, insbesondere von 100 bis 400 kDa, bzw. 200 bis 300 kDa, werden besagte Stärken bevorzugt einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, extruder, Stator/Rotor-Maschinen und/oder Rührwerke.

Die oxidative Spaltung mittels Wasserstoffperoxid ist erfindungsgemäß bevorzugt. Zu diesem Zweck wird beispielsweise die mit Propylenoxid modifizierte Stärke in Wasser gegeben, auf 50 bis 70 °C erhitzt, Wasserstoffperoxid zugefügt und bei 70 bis 85°C für 2 bis 5 Stunden gerührt.

Der Gehalt an Propylenoxid der Stärke wirkt sich auf die Feinabstimmung des Frisurenhalts mit der Frisurenflexibilität und der Stabilität der kosmetischen Mittel aus. Es zeigte sich, dass die Parameter weiter optimiert werden, wenn die mit Propylenoxid modifizierte Stärke - bezogen auf das Gewicht der besagten Stärke - bevorzugt einen Propylenoxidgehalt von 1 bis 20 Gew.-%, insbesondere von 5 bis 15 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder 4 bis 6 Gew.-%, aufweist. Der Propylenoxidgehalt lässt sich beispielsweise nach Durchführung einer Hodges-Spaltung mit der Methode gemäß DIN EN 13268 bestimmen.

Im Rahmen einer bevorzugten Ausführungsform ist die mit Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Ferner hat es sich erwiesen, dass solche kosmetischen Mittel sich im Sinne der Erfindung hervorragend eignen, in denen die mit Propylenoxid modifizierte Stärke in einer 43 Gew.-%-igen wässrigen Lösung eine Viskosität eine bevorzugte Viskosität im Bereich von 150 bis 1500000 mPa·s (Brookfield Viskosimeter, Spindel #7 bei 20°C und 20 U/min) aufweist. Besonders geeignete mit Propylenoxid modifizierte Stärken weisen Viskositäten von 10000 bis 200000 mPa·s, besonders bevorzugt von 25000 bis 180000 mPa·s, (jeweils gemessen unter den zuvor genannten Bedingungen) auf.

Eine erfindungsgemäß besonders bevorzugte mit Propylenoxid modifizierte Stärke ist unvernetzt, weist ein mittleres Molekulargewicht (Gewichtsmittel) 700 bis 1000 kDa, auf und hat - bezogen auf das Gewicht der modifizierten Stärke - einen Propylenoxidgehalt von 1 bis 20 Gew.-%, insbesondere von 5 bis 15 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke.

Eine erfindungsgemäß besonders bevorzugte mit Propylenoxid modifizierte Stärke ist unvernetzt, weist ein mittleres Molekulargewicht (Gewichtsmittel) von 700 bis 1000 kDa, auf und hat - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke. Eine erfindungsgemäß ganz besonders bevorzugte mit Propylenoxid modifizierte Kartoffelstärke ist unvernetzt, weist ein mittleres Molekulargewicht (Gewichtsmittel) von 700 bis 1000 kDa, auf und hat - bezogen auf das Gewicht der modifizierten Kartoffelstärke - einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%.

Es ist erfindungsgemäß bevorzugt, wenn das kosmetisches Mittel die mit Propylenoxid modifizierte Stärke in einer Menge von 0,1 Gew.-% bis 80 Gew.-%, insbesondere von 0,5 Gew.-% bis 40 Gew.-%, ganz besonders bevorzugt von 2 Gew.-% bis 10 Gew.-%, jeweils bezogen auf das Gewicht des Mittels, enthält. Diese Gewichtsangaben sind insbesondere dann bevorzugt, wenn das erfindungsgemäße Mittel in Form eines Gels oder einer Creme vorliegt.

Wenn das erfindungsgemäße Mittel in Form eines Schaums, insbesondere in Form eines Aerosolschaums *(vide infra),* vorliegt, enthält es die mit Propylenoxid modifizierte Stärke bevorzugt in einer Menge von 0,1 Gew.-% bis 20 Gew.-%, insbesondere von 1,0 Gew.-% bis 10 Gew.-%, wiederum besonders bevorzugt von 2 Gew.-% bis 8 Gew.-%, ganz besonders bevorzugt von 3 Gew.-% bis 6 Gew.-%, jeweils bezogen auf das Gewicht des Mittels.

Das erfindungsgemäße kosmetische Mittel kann, wenn es in Form eines Gels oder einer Creme vorliegt, zusätzlich mindestens ein filmbildendes und/oder festigendes Polymer enthalten. Bevorzugt gilt die Maßgabe, dass die Gewichtsmenge des zusätzlichen filmbildenden und/oder festigenden Polymers kleiner ist, als die enthaltene Gewichtsmenge der mit Propylenoxid modifizierten Stärke. Bevorzugt enthält das erfindungsgemäße Mittel die mit Propylenoxid modifizierte Stärke und die zusätzlichen filmbildenden und/oder festigenden Polymere in einem Gewichtsverhältnisbereich von 1 zu 1.5 bis 1 zu 10, insbesondere von 1 zu 2 bis 1 zu 8.

Das erfindungsgemäße kosmetische Mittel kann, wenn es in Form eines Schaums, insbesondere in Form eines Aerosolschaums (*vide infra)* vorliegt, zusätzlich mindestens ein filmbildendes und/oder festigendes Polymer enthalten. Bevorzugt gilt dabei die Maßgabe, dass es die mit Propylenoxid modifizierte Stärke und die zusätzlichen filmbildenden und/oder festigenden Polymere in einem Gewichtsverhältnisbereich von 4 zu 1 bis 1 zu 4, insbesondere von 3 zu 1 bis 1 zu 3.

Unter Polymeren werden erfindungsgemäß Verbindungen verstanden, die aus einer Vielzahl von Molekülen aufgebaut sind, in denen eine Art oder mehrere Arten von Atomen oder Atomgruppierungen (sogenannte konstitutive Einheiten, Grundbausteine oder Wiederholungseinheiten) wiederholt aneinander gereiht sind und die ein Molekulargewicht von wenigstens 10000 g/mol besitzen. Die Polymere werden durch Polyreaktion erhalten, wobei letztere künstlich (d.h. synthetisch) oder natürlich erfolgen kann.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nichtionisch, permanent kationisch oder temporär kationisch geladen sein.

Besonders bevorzugt eignen sich erfindungsgemäß für diese Ausführungsform solche Mittel, die zusätzlich mindestens ein filmbildendes und/oder festigende Polymer enthalten, welches aus mindestens einem Polymer der Gruppe ausgewählt wird, die gebildet wird aus

- nichtionischen Polymeren auf Basis ethylenisch ungesättigter Monomere, insbesondere aus
  - Homopolymeren des N-Vinylpyrrolidons,
  - nichtionischen Copolymeren des N-Vinylpyrrolidons,
  - Homopolymeren und nichtionischen Copolymeren des N-Vinylcaprolactams,
  - Copolymeren des (Meth)acrylamids,
  - Polyvinylalkohol, Polyvinylacetat,
- Chitosan und Derivaten des Chitosans,
- kationischen Cellulosederivaten,
- kationischen Copolymeren des 3-($C_1$ bis $C_6$)-Alkyl-1-vinyl-imidazoliniums,
- Copolymeren enthaltend die Struktureinheit der Formel (M-1)

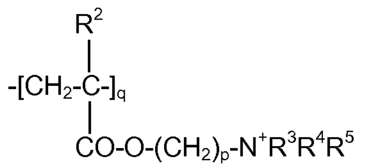 $X^-$ (M-1)

in der $R^2$= -H oder $-CH_3$ ist, $R^3$, $R^4$ und $R^5$ unabhängig voneinander ausgewählt sind aus ($C_1$ bis $C_4$)-Alkyl-, ($C_1$ bis $C_4$)-Alkenyl- oder ($C_2$ bis $C_4$)-Hydroxyalkylgruppen, p = 1, 2, 3 oder 4, q eine natürliche Zahl und $X^-$ ein physiologisch verträgliches organisches oder anorganisches

Anion ist,

- anionischen Polymeren, welche Carboxylat- und/oder Sulfonatgruppen aufweisen,
- amphoteren Poymeren,
- anionischen Polyurethanen.

Bevorzugt als zusätzliches filmbildendes und/oder festigendes Polymer geeignete nichtionische Polymere auf Basis ethylenisch ungesättigter Monomere, sind solche nichtionischen Polymere, welche mindestens eine der nachfolgenden Struktureinheiten enthalten

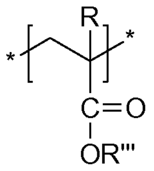 (M2) 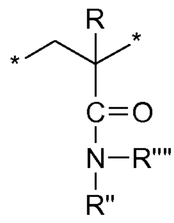 (M3) 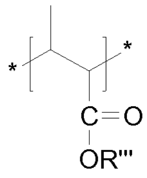 (M4)

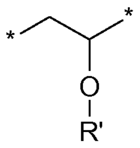 (M5) 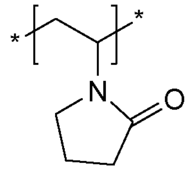 (M6) 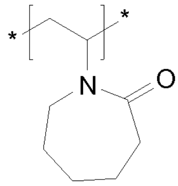 (M7)

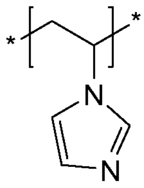 (M8)

worin

R steht für ein Wasserstoffatom oder eine Methylgruppe,
R' steht für ein Wasserstoffatom oder eine ($C_1$ bis $C_4$)-Acylgruppe,
R" und R"" stehen unabhängig voneinander für eine ($C_1$ bis $C_7$)-Alkylgruppe oder ein Wasserstoffatom
R'" steht für eine lineare oder verzweigte ($C_1$ bis $C_4$)-Alkylgruppe oder eine ($C_2$ bis $C_4$)-Hydroxyalkylgruppe.

Bevorzugte, nichtionische filmbildende und/oder nichtionische haarfestigende Polymere sind Homo-oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, wobei jeweils die Alkylgruppen dieser Monomere aus ($C_1$ bis $C_3$)-Alkylgruppen ausgewählt werden.

Für die erfindungsgemäßen Mittel besonders geeignete nichtionische Polymere auf Basis ethylenisch ungesättigter Monomere enthalten mindestens eine der nachfolgenden Struktureinheiten

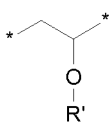 (M5) 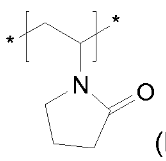 (M6) 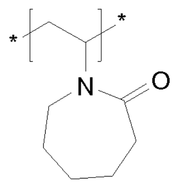 (M7) 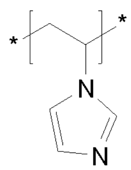 (M8)

worin R' steht für ein Wasserstoffatom oder eine ($C_1$- bis $C_{30}$)-Acylgruppe, insbesondere für ein Wasserstoffatom oder eine Acetylgruppe.

Geeignet sind insbesondere Homopolymere des Vinylcaprolactams oder des Vinylpyrrolidons (wie beispielsweise Luviskol® K 90 oder Luviskol® K 85 der Firma BASF SE), Copolymerisate aus Vinylpyrrolidon und Vinylacetat (wie sie beispielsweise unter dem Warenzeichen Luviskol® VA 37, Luviskol® VA 55, Luviskol® VA 64 und Luviskol® VA 73 von der Firme BASF SE vertrieben werden), Terpolymere aus Vinylpyrrolidon, Vinylacetat und Vinylpropionat, Polyacrylamide (wie beispielsweise Akypomine® P 191 von der Firma CHEM-Y), Polyvinylalkohole (die beispielsweise unter den Handelsbezeichnungen Elvanol® von Du Pont oder Vinol® 523/540 von der Firma Air Products vertrieben werden), Terpolymere aus Vinylpyrrolidon, Methacrylamid und Vinylimidazol (wie beispielsweise Luviset® Clear der Firma BASF SE).

Neben den auf ethylenisch ungesättigten Monomeren basierenden nichtionischen Polymeren eignen sich weiterhin zur bevorzugten Ausführung der technischen Lehre nichtionische Cellulosederivate als filmbildende und/oder festigende Polymere, die bevorzugt ausgewählt werden aus Methylcellulose und insbesondere aus Celluloseether, wie Hydroxypropylcellulose (z. B. Hydroxypropylcellulose mit einem Molekulargewicht von 30.000 bis 50.000 g/mol, welche

beispielsweise unter der Handelsbezeichnung Nisso SI® von der Firma Lehmann & Voss, Hamburg, vertrieben wird), Hydroxyethylcellulose, wie sie beispielsweise unter den Warenzeichen Culminal® und Benecel® (AQUALON) und Natrosol®-Typen (Hercules) vertrieben werden.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes kationisches filmbildendes und/oder kationisches festigendes Polymer ist mindestens ein kationisches filmbildendes und/oder kationisches festigendes Polymer, das mindestens ein Strukturelement der Formel (M9) und zusätzlich mindestens ein Strukturelement der Formel (M10) enthält

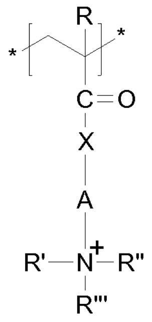 (M9)

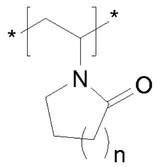 (M10)

worin

R für ein Wasserstoffatom oder eine Methylgruppe steht,
R’, R" und R" unabhängig voneinander stehen für eine ($C_1$ bis $C_{30}$)-Alkylgruppe,
X steht für ein Sauerstoffatom oder eine Gruppe NH,
A steht für eine Ethan-1,2,-diylgruppe oder eine Propan-1,3-diylgruppe,
n 1 oder 3 bedeutet.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als

- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-WasserGemisch) von der Firma ISP vertrieben wird.

Weiterhin werden die kationischen filmbildenden und/oder kationischen festigenden Polymere erfindungsgemäß besonders bevorzugt aus kationischen, quaternisierten Cellulose-Derivaten ausgewählt.

Ferner eignen sich als filmbildendes und/oder festigendes Polymere bevorzugt kationische, quaternisierte Cellulosederivate.

Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.

Als im Sinne der Erfindung besonders bevorzugt verwendbare kationische Polymere dienen weiterhin solche kationischen filmbildenden und/oder kationischen festigenden Copolymere, die mindestens ein Strukturelement der Formel (M11)

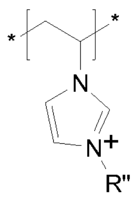

(M11)

worin R" für eine ($C_1$ bis $C_4$)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches filmbildendes und/oder kationisches festigendes Polymer, mindestens ein Copolymer (c1) enthalten ist, das neben mindestens einem Strukturelement der Formel (M11) zusätzlich ein Strukturelement der Formel (M6) umfasst

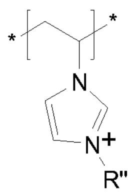

(M11)

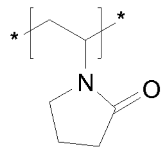

(M6).

worin R" für eine ($C_1$ bis $C_4$)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Besonders bevorzugte erfindungsgemäße Mittel enthalten ein Copolymer (c1), insbesondere der Formel (Poly1 das Molmassen innerhalb eines bestimmten Bereiches aufweist. Hier sind erfindungsgemäße Mittel bevorzugt, bei denen das Copolymer (c1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (M7) aufweisen

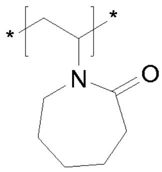

(M7).

Weitere besonders bevorzugte erfindungsgemäße Mittel sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

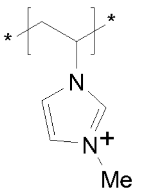 (M11-a) 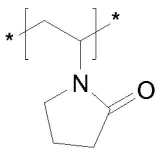 (M6) 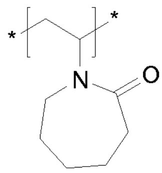 (M7)

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Besonders bevorzugte erfindungsgemäße Mittel enthalten ein Copolymer (c2), das Molmassen innerhalb eines bestimmten Bereiches aufweist. Hier sind erfindungsgemäße Mittel bevorzugt, bei denen das Copolymer (c2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

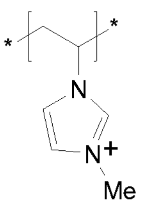 (M11-a) 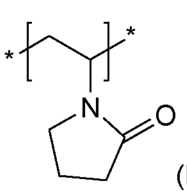 (M6) 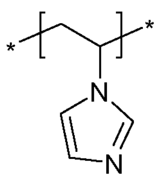 (M8) 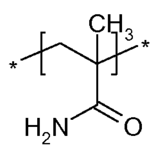 (M12).

Auch hierbei ist besonders bevorzugt, wenn die Copolymere (c3) neben Polymereinheiten, die aus dem Einbau

der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Besonders bevorzugte erfindungsgemäße Mittel enthalten ein Copolymer (c3), das Molmassen innerhalb eines bestimmten Bereiches aufweist. Hier sind erfindungsgemäße Mittel bevorzugt, bei denen das Copolymer (c3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren mit mindestens einem Strukturelement der obigen Formel (M11-a), gelten als bevorzugt:

- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat® Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat® Care oder Luviquat® Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)),

sowie Gemische aus diesen Polymeren.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Zu diesen Polymeren gehören beispielsweise Chitosane. Chitosan und/oder Chitosanderivate gelten als ganz besonders bevorzugt geeignete filmbildende und/oder festigende Polymere im Sinne der vorliegenden Erfindung.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typischen kationischen Biopolymeren kommen im Sinne der Erfindung auch kationisch derivatisierte Chitosane (wie z. B. Quaternierungsprodukte) oder alkoxylierte Chitosane in Frage.

Erfindungsgemäß bevorzugte Mittel sind dadurch gekennzeichnet, daß sie als Chitosanderivat(e) Neutralisationsprodukte von Chitosan mit mindestens einer Säure, ausgewählt aus Milchsäure, Pyrrolidoncarbonsäure, Nicotinsäure, Hydroxyisobuttersäure, Hydroxyisovaleriansäure enthaltend oder Gemische dieser Neutralisationsprodukte umfassen.

Geeignete Chitosan(derivate) sind beispielsweise unter den Handelsbezeichnungen Hydagen® CMF (1 Gew.-% Aktivsubstanz in wässriger Lösung mit 0.4 Gew.-% Glykolsäure, Molekulargewicht 500000 bis 5000000 g/mol Cognis),

Hydagen® HCMF (Chitosan (zu 80 % deacetyliert), Molekulargewicht 50000 bis 1000000 g/mol, Cognis), Kytamer® PC (80 Gew.-% Aktivsubstanz an Chitosan pyrolidoncarboxylat (INCI-Bezeichnung: Chitosan PCA), Amerchol) und Chitolam® NB/101 im Handel frei verfügbar.

Das Chitosan bzw. dessen Derivate sind in den erfindungsgemäßen Mitteln bevorzugt in einer Menge von 0,01 Gew.-% bis 20 Gew.-%, besonders bevorzugt von 0,01 Gew.-% bis 10,0 Gew.-%, ganz besonders bevorzugt von 0,1 Gew.-% bis 1 Gew.-%, jeweils bezogen auf das Gewicht des erfindungsgemäßen Mittels, enthalten.

Als im Sinne der Erfindung bevorzugt geeignete temporär kationische Polymere gelten gleichfalls solche, die mindestens eine Struktureinheit der Formeln (M1-1) bis (M1-8) aufweisen

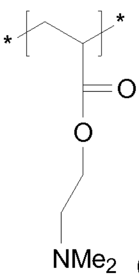 (M1-1) 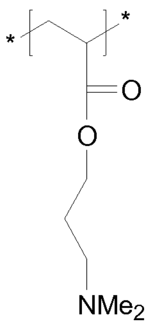 (M1-2) 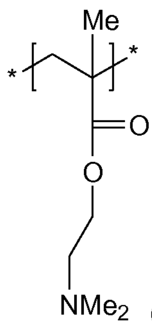 (M1-3) 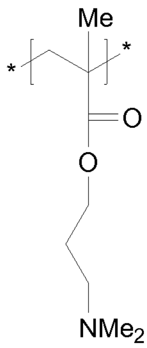 (M1-4)

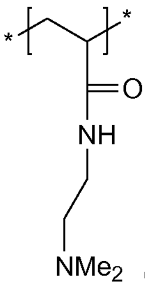 (M1-5) 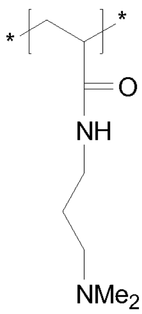 (M1-6) 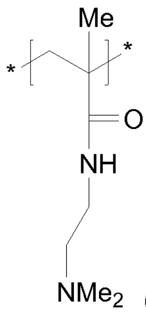 (M1-7) 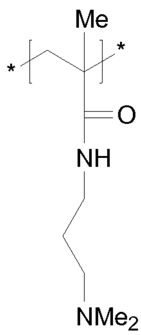 (M1-8).

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten,

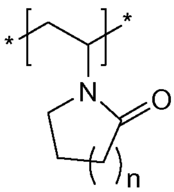 (M10)

worin

n 1 oder 3 bedeutet.

Dabei gilt wiederum die Gruppe der Polymere

- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix® VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex® SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Di-

methylaminoethyl Methacrylate Copolymer, Alcohol, Lauryl Pyrrolidone (ISP)),

- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),

als bevorzugte Liste zur Auswahl mindestens eines oder mehrerer Polymere daraus.

Weiterhin können als filmbildende und/oder festigende Polymere mindestens ein anionisches filmbildendes und/oder anionisches festigendes Polymer eingesetzt werden.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Weitere bevorzugt einsetzbare anionische Polymere werden ausgewählt aus der Gruppe, die gebildet wird, aus

- Copolymeren aus Vinylacetat und Crotonsäure (wie sie beispielsweise als Handelsprodukt Aristoflex® A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex® Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichung Polyurethane-1 von der Firma BASF SE vertrieben wird).

Die zusätzlichen Polymere sind bevorzugt in einer Menge von 0,1 Gew.-% bis 15 Gew.-%, insbesondere von 0,5 Gew.-% bis 10 Gew.-%, jeweils bezogen auf das Gewicht des Mittels, enthalten.

Die erfindungsgemäßen Mittel können als filmbildendes und/oder festigendes Polymer auch mindestens ein amphoteres Polymer enthalten. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefaßt, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Die amphoteren Polymere sind in den erfindungsgemäßen Mitteln bevorzugt in Mengen von 0,01 Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Als bevorzugte Polymere amphotere Polymere eignen sich Copolymere, die gebildet werden aus

- mindestens einem Monomer (Mo1) ausgewählt aus Acrylsäure, Methacrylsäure, Acrylsäurealkylestern und Methacrylsäurealkylestern, und
- mindestens einem amphoteren Monomer (Mo2) ausgewählt aus (Meth)acryloylalkyl-aminoxiden der Formel (Mo2)

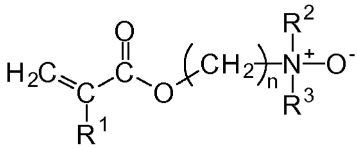

(Mo2)

worin

- $R^1$ für H oder $CH_3$,
- $R^2$ und $R^3$ jeweils unabhängig voneinander für gegebenenfalls verzweigtes $C_{1-10}$-Alkyl und
- n für eine ganze Zahl von 1 bis 20 steht.

Bevorzugt steht n der Formel (Mo2) für 2 oder 3.
$R^2$ und $R^3$ der Formel (Mo2) stehen unabhängig voneinander bevorzugt für Methyl oder Ethyl.

In einer bevorzugten Variante dieser Ausführungsform enthält das erfindungsgemäße Mittel zusätzlich mindestens ein Copolymer, das gebildet ist aus

- mindestens zwei Monomeren (Mo1 wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
- als Monomer (Mo2) Methacryloylethyldimethylaminoxid.

Auch diese Copolymere sind bekannt und beispielsweise unter der Bezeichnung Diaformer Z-632 von der Firma Clariant erhältlich, wobei der Einsatz von Diaformer Z-632 besonders bevorzugt ist.

In einer weiteren bevorzugten Variante dieser Ausführungsform enthält das erfindungsgemäße Mittel mindestens ein Copolymer, das gebildet ist aus

- mindestens drei Monomeren (Mo1 wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, das zweite Monomer ausgewählt ist aus Acrylsäurelaurylester und Methacrylsäurelaurylester, und das dritte Monomer ausgewählt ist aus Acrylsäurestearylester und Methacrylsäurestearylester, und
- als Monomer (Mo2) Methacryloylethyldimethylaminoxid.

Entsprechende Copolymere sind ebenfalls bekannt und beispielsweise unter den Bezeichnungen Diaformer Z-611, Diaformer Z-612, Diaformer Z-613, Diaformer Z-631, Diaformer Z-633, Diaformer Z-651, Diaformer Z-711 N, Diaformer Z-712N und Diaformer Z-731 N von der Firma Clariant erhältlich, wobei der Einsatz von Diaformer Z-712N und Diaformer Z-651 besonders bevorzugt ist.

Erfindungsgemäß ganz besonders bevorzugte kosmetische Mittel gehorchen mindestens einer der folgenden Ausführungsformen A) bis I):

A):
Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke mit einem mittleren Molekulargewicht (Gewichtsmittel) von 50 bis 2500 kDa, bevorzugt von 100 bis 2000 kDa, bevorzugt von 500 bis 1800 kDa, besonders bevorzugt von 700 bis 1000 kDa, welche - bezogen auf das Gewicht besagter Stärke - einen Propylenoxidgehalt von 1 bis 20 Gew.-% aufweist.
B):
Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke mit einem mittleren Molekulargewicht (Gewichtsmittel) von 50 bis 2500 kDa, bevorzugt von 100 bis 2000 kDa, bevorzugt von 500 bis 1800 kDa, besonders bevorzugt von 700 bis 1000 kDa, welche - bezogen

auf das Gewicht besagter Stärke - einen Propylenoxidgehalt von 8,0 bis 12,0 Gew.-% aufweist.

C):

Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke mit einem mittleren Molekulargewicht (Gewichtsmittel) von 50 bis 2500 kDa, bevorzugt von 100 bis 2000 kDa, bevorzugt von 500 bis 1800 kDa, besonders bevorzugt von 700 bis 1000 kDa, welche - bezogen auf das Gewicht besagter Stärke - einen Propylenoxidgehalt von 9,5 bis 10,5 Gew.-% oder 4 bis 6 Gew.-% aufweist.

D):

Kosmetisches Mittel zur temporären Umformung keratinischer Fasern, insbesondere menschlicher Haare, enthaltend in einem in Form eines dispersen Systems vorliegenden, kosmetischen Träger

(a) mindestens eine mit Propylenoxid modifizierte Stärke mit einem mittleren Molekulargewicht (Gewichtsmittel) von 100 bis 2000 kDa, bevorzugt von 500 bis 1800 kDa, besonders bevorzugt von 700 bis 1000 kDa, welche - bezogen auf das Gewicht besagter Stärke - einen Propylenoxidgehalt von 1 bis 20 Gew.-% aufweist und
(b) mindestens ein zusätzliches filmbildendes und/oder festigendes Polymer.

E):

Kosmetisches Mittel zur temporären Umformung keratinischer Fasern, insbesondere menschlicher Haare, enthaltend in einem in Form eines dispersen Systems vorliegenden, kosmetischen Träger

(a) mindestens eine mit Propylenoxid modifizierte Stärke mit einem mittleren Molekulargewicht (Gewichtsmittel) von 100 bis 2000 kDa, bevorzugt von 500 bis 1800 kDa, besonders bevorzugt von 700 bis 1000 kDa, welche - bezogen auf das Gewicht besagter Stärke - einen Propylenoxidgehalt von 4 bis 12 Gew.-% aufweist und
(b) mindestens ein zusätzliches filmbildendes und/oder festigendes Polymer.

F):

Kosmetisches Mittel zur temporären Umformung keratinischer Fasern, insbesondere menschlicher Haare, enthaltend in einem in Form eines dispersen Systems vorliegenden, kosmetischen Träger

(a) mindestens eine mit Propylenoxid modifizierte Stärke mit einem mittleren Molekulargewicht (Gewichtsmittel) von 100 bis 2000 kDa, bevorzugt von 500 bis 1800 kDa, besonders bevorzugt von 700 bis 1000 kDa, welche - bezogen auf das Gewicht besagter Stärke - einen Propylenoxidgehalt von 9,5 bis 10,5 Gew.-% oder 4 bis 6 Gew.-% aufweist und
(b) mindestens ein zusätzliches filmbildendes und/oder festigendes Polymer.

G):

Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke, welche ein mittleres Molekulargewicht (Gewichtsmittel) von 100 bis 2000 kDa, bevorzugt von 500 bis 1800 kDa, besonders bevorzugt von 700 bis 1000 kDa sowie - bezogen auf das Gewicht besagter Stärke - einen Propylenoxidgehalt von 1 bis 20 Gew.-% aufweist.

H):

Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke welche ein mittleres Molekulargewicht (Gewichtsmittel) von 100 bis 2000 kDa, bevorzugt von 500 bis 1800 kDa, besonders bevorzugt von 700 bis 1000 kDa sowie - bezogen auf das Gewicht besagter Stärke - einen Propylenoxidgehalt von 4 bis 12 Gew.-% aufweist.

I):

Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke welche ein mittleres Molekulargewicht (Gewichtsmittel) von 100 bis 2000 kDa, bevorzugt von 500 bis 1800 kDa, besonders bevorzugt von 700 bis 1000 kDa sowie - bezogen auf das Gewicht besagter Stärke - einen Propylenoxidgehalt von 9,5 bis 10,5 Gew.-% oder 4 bis 6 Gew.-% aufweist.

Im Rahmen der zuvor genannten Ausführungsformen gelten selbstverständlich *mutatis mutandis* die jeweiligen als bevorzugt gekennzeichneten Merkmale des erfindungsgemäß Mittels, wie insbesondere die Einsatzmengen.

Zur Verbesserung der erfindungsgemäßen Effekte eignet sich bevorzugt der weitere Zusatz mindestens einer Verbindung der Formel (I),

$$HO\text{-}CH_2\text{-}(CHOH)_n\text{-}CH_2\text{-}OH \qquad (I)$$

worin n eine ganze Zahl von 1 bis 4 bedeutet.

Besonders effektvolle zeigen sich die erfindungsgemäßen Mittel, wenn sie als Verbindungen der Formel (I) Glycerin und/oder Sorbitol enthalten.

Ferner erwies sich eine Einsatzmenge der Verbindungen der Formel (I) im Bereich von 0,2 bis 10 Gew.-%, insbesondere von 0,5 bis 7 Gew.-% als vorteilhaft.

Es hat sich als bevorzugt herausgestellt, zusätzlich mindestens ein nichtionisches Tensid einzusetzen. Diese Tenside können erfindungsgemäß bereits emulgierende Wirkung haben.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare oder verzweigte Fettalkohole mit 8 bis 30 Kohlenstoff-Atomen, an Fettsäuren mit 8 bis 30 Kohlenstoff-Atomen und an Alkylphenole mit 8 bis 15 C-Atomen in der Alkylgruppe,
- Anlagerungsprodukte von 2 bis 20 Einheiten Glyzerin an lineare oder verzweigte Fettalkohole mit 8 bis 30 Kohlenstoff-Atomen in der Alkylgruppe, an lineare oder verzweigte Fettsäuren mit 8 bis 30 Kohlenstoff-Atomen in der Alkylgruppe, wie beispielsweise die unter den Verkaufsbezeichnungen Dermofeel® G 10 LW (Straetmans Chemische Produkte) erhältlichen Typen,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 Kohlenstoff -Atomen, an Fettsäuren mit 8 bis 30 C-Atomen und an Alkylphenole mit 8 bis 15 Kohlenstoff -Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad (E4\text{-}I)$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad (E4\text{-}II)$$

  in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Bevorzugt sind Alkyloligoglucoside auf Basis von gehärtetem $C_{12/14}$-Kokosalkohol mit einem DP von 1 bis 3.

Darunter sind wiederum solche nichtionischen Tenside besonders bevorzugt zum Einsatz in dem erfindungsgemäßen Mittel geeignet, die ausgewählt werden aus

- Anlagerungsprodukten von 2 bis 20 Einheiten Glyzerin an lineare oder verzweigte Fettalkohole mit 8 bis 30 Kohlenstoff-Atomen in der Alkylgruppe,
- Anlagerungsprodukten von 2 bis 20 Einheiten Glyzerin an lineare oder verzweigte Fettsäuren mit 8 bis 30 Kohlenstoff-Atomen in der Alkylgruppe,

- Anlagerungsprodukte von 5 bis 60 Mol Ethylenoxid an Rizinusöl und gehärtetes Rizinusöl,
- Zuckertensiden vom Typ der Alkyl- und Alkenyloligoglykoside gemäß obiger Formel (E4-II),
- Mischungen aus den vorgenannten Tensiden.

Die nichtionischen Tenside sind bevorzugt in einer Menge von 0,005 Gew.-% bis 10 Gew.-%, insbesondere von 0,01 bis 2 Gew.-%, jeweils bezogen auf das Gewicht des erfindungsgemäßen Mittels, in dem erfindungsgemäßen Mittel enthalten.

Ferner enthalten die erfindungsgemäßen Mittel bevorzugt zusätzlich mindestens ein Pflanzenextrakt. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Erfindungsgemäß sind vor allem die Extrakte aus Bambus, Leinsamen, Seerose, grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Der zusätzliche Pflanzenextrakt ist bevorzugt in dem erfindungsgemäßen Mittel in einer Menge von 0,05 Gew.-% bis 1,0 Gew.-%, insbesondere von 0,1 Gew.-% bis 0,5 Gew.-%, jeweils bezogen auf das Gewicht des kosmetischen Mittels, enthalten.

Es ist erfindungsgemäß bevorzugt - insbesondere wenn das erfindungsgemäße Mittel als Creme formuliert ist - dass das erfindungsgemäße kosmetische Mittel zusätzlich mindestens eine Ölphase enthält.

Unter einer Ölphase versteht sich erfindungsgemäß eine bei 20°C flüssige Phase, die sich bei 20°C zu weniger als 1 g in 100 g Wasser löst.

Die Ölphase besitzt bevorzugt eine Viskosität bis zu 1000 mPas, (Brookfield, RVDV II+, 20°C, 20 Umdrehungen pro Minute, Spindel Nr. 1).

In einer bevorzugten Ausführungsform wird das Öl der Ölphase ausgewählt aus mindestens einem Öl der Gruppe, die gebildet wird aus

pflanzliche Öle,

tierische Öle,

Esterölen,

flüssige Fettsäuren und/oder deren Mono,- Di- und Trifettsäureester von gesättigten und/oder ungesättigten linearen und/oder verzweigten $C_6$- bis $C_{22}$-Fettsäuren mit Glycerin.

Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Ricinusöl, Sesamöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Esteröle werden ausgewählt aus Estern von $C_6$ - $C_{30}$ - Fettsäuren mit $C_2$ - $C_{30}$-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

Bevorzugt als Öl in der Ölphase verwendbare Mono,- Di- und Trifettsäureester von gesättigten und/oder ungesättigten linearen und/oder verzweigten Fettsäuren mit Glycerin sind die insbesondere Triglyceridester aus Caprinsäure und Caprylsäure (INCI-Bezeichnung: Caprylic/Capric Triglyceride) beispielsweise erhältlich als Handelsprodukt der Firma Cognis unter der Bezeichnung Myritol® 312.

Die zusätzliche Ölphase ist bevorzugt in dem erfindungsgemäßen Mittel in einer Menge von 0,05 Gew.-% bis 25 Gew.-%, insbesondere von 0,1 Gew.-% bis 20 Gew.-%, jeweils bezogen auf das Gewicht des kosmetischen Mittels, enthalten.

Weiterhin eignen sich erfindungsgemäße Mittel, die zusätzlich mindestens einen Fettstoff enthalten.

Unter einem Fettstoff werden erfindungsgemäß solche Verbindungen verstanden, die bei 20°C zu weniger als 1 g in 100 g Wasser löslich sind.

Bevorzugt wird der Fettstoff ausgewählt aus mindestens einem Fettstoff der Gruppe, die gebildet wird aus Candelillawachs, Sheabutter, Carnaubawachs, Bienenwachs, Kokosfett, $C_{12}$ bis $C_{20}$-Fettsäuren (insbesondere Palmitinsäure, Stearinsäure)

Der zusätzliche Fettstoff ist bevorzugt in dem erfindungsgemäßen Mittel in einer Menge von 0,05 Gew.-% bis 35 Gew.-%, insbesondere von 1 Gew.-% bis 20 Gew.-%, jeweils bezogen auf das Gewicht des kosmetischen Mittels, enthalten.

Folgende erfindungsgemäße kosmetische Mittel der Ausführungsformen A-1) bis I-1) umfassend mindestens ein Öl und/oder einen Fettstoff sind erfindungsgemäß bevorzugt:

A-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke mit einem mittleren Molekulargewicht (Gewichtsmittel) von bis 1000 kDa, welche - bezogen auf das Gewicht besagter Stärke - einen Propylenoxidgehalt von 1 bis 20 Gew.-% aufweist.

B-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 8,0 bis 12,0 Gew.-% aufweist.

C-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 9,5 bis 10,5 Gew.-% oder 4 bis 6 Gew.-% aufweist.

D-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

(a) mindestens eine mit Propylenoxid modifizierte Stärke mit einem mittleren Molekulargewicht (Gewichtsmittel) von 700 bis 1000 kDa, welche - bezogen auf das Gewicht besagter Stärke-einen Propylenoxidgehalt von 1 bis

20 Gew.-% aufweist und
(b) mindestens ein zusätzliches filmbildendes und/oder festigendes Polymer.

E-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

(a) mindestens eine mit Propylenoxid modifizierte Stärke mit einem mittleren Molekulargewicht (Gewichtsmittel) von 700 bis 1000 kDa, welche - bezogen auf das Gewicht besagter Stärke-einen Propylenoxidgehalt von 4 bis 12 Gew.-% aufweist und
(b) mindestens ein zusätzliches filmbildendes und/oder festigendes Polymer.

F-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

(a) mindestens eine mit Propylenoxid modifizierte Stärke mit einem mittleren Molekulargewicht (Gewichtsmittel) von 700 bis 1000 kDa, welche - bezogen auf das Gewicht besagter Stärke-einen Propylenoxidgehalt von 9,5 bis 10,5 Gew.-% oder 4 bis 6 Gew.-% aufweist und
(b) mindestens ein zusätzliches filmbildendes und/oder festigendes Polymer.

G-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke, welche ein mittleres Molekulargewicht (Gewichtsmittel) von 700 bis 1000 kDa sowie - bezogen auf das Gewicht besagter Stärke - einen Propylenoxidgehalt von 1 bis 20 Gew.-% aufweist.
H-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke, welche ein mittleres Molekulargewicht (Gewichtsmittel) von 700 bis 1000 kDa sowie - bezogen auf das Gewicht besagter Stärke - einen Propylenoxidgehalt von 4 bis 12 Gew.-% aufweist.
I-1):
Kosmetisches Mittel zur temporären Umformung keratinischer Fasern, insbesondere menschlicher Haare, enthaltend in einem in Form eines dispersen Systems vorliegenden, kosmetischen Träger umfassend

- Wasser,
- mindestens ein nichtionisches Tensid und
- mindestens ein Öl und/oder einen Fettstoff,

mindestens eine mit Propylenoxid modifizierte Stärke, welche ein mittleres Molekulargewicht (Gewichtsmittel) von 700 bis 1000 kDa sowie - bezogen auf das Gewicht besagter Stärke - einen Propylenoxidgehalt von 9,5 bis 10,5 Gew.-% oder 4 bis 6 Gew.-% aufweist.

Das erfindungsgemäße kosmetische Mittel kann - bevorzugt unter Einhaltung der Maxime, nur solche Rohstoffe einzusetzen, die nicht ihren Ursprung in fossilen Brennstoffen haben - weiterhin zusätzliche Hilfs- und Zusatzstoffe enthalten.

Als geeignete Hilfs- und Zusatzstoffe sind insbesondere Pflegestoffe zu nennen.

Als Pflegestoff kann ein kationisches Tensid eingesetzt werden. Bevorzugt sind dabei kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Da sich der Zusatz oberflächenaktiver Substanzen jedoch negativ auf die hydrophoben Eigenschaften des hydrophobierten Siliciumdioxids und damit auf die Stabilität des erfindungsgemäßen kosmetischen Mittels auswirken kann, ist die Menge an pflegendem Tensid sorgfältig auf die Gesamtzusammensetzung abzustimmen. Vorzugsweise wird auf den Zusatz tensidischer Bestandteile verzichtet.

Als Pflegestoff eignen sich ebenfalls pflegende Polymere.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein mittleres Molekulargewicht (Gewichtsmittel) von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI-Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Bevorzugt sind die kationischen Proteinhydrolysate und -derivate auf pflanzlicher Basis.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin $B_5$ (Pantothensäure, Panthenol und Pantolacton).

Als Pflegestoff eignen sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten,

bevorzugt ist. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda), Crosilk® (Croda) oder Crotein® (Croda) vertrieben.

Selbstverständlich umfasst die erfindungsgemäße Lehre alle isomeren Formen, wie cis - trans-Isomere, Diastereomere und chirale Isomere.

Erfindungsgemäß ist es auch möglich, eine Mischung aus mehreren Proteinhydrolysaten einzusetzen.

Weiterhin sind als Pflegestoff Lipide und Ölkörper, beispielsweise pflanzliche Öle, flüssige Paraffinöle, Isoparaffinöle, synthetische Kohlenwasserstoffe und Esteröle, Enzyme und Perlenextrakte geeignet.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den erfindungsgemäßen kosmetischen Mitteln zusätzlich mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul®MS 40; Uvasorb®S 5) genannt.

In einer besonderen Ausführungsform enthält das erfindungsgemäße kosmetische Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Weiterhin können die erfindungsgemäßen kosmetischen Mittel Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von $\omega$-Aminosäuren wie $\omega$-Aminocapronsäure als Alkalisierungsmittel ist möglich.

Vorzugsweise werden die erfindungsgemäßen Mittel als Creme oder Gel oder Pumpschaum oder Aerosolschaum ausgestaltet.

Falls die erfindungsgemäßen Mittel in Form einer Creme oder eines Gels vorliegen, ist es erfindungsgemäß bevorzugt, zusätzlich mindestens ein polymeres Verdickungsmittel zuzusetzen. Dieses Verdickungsmittel ist von den besagten mit Propylenoxid modifizierten Stärken verschieden. Sie können beispielsweise ausgewählt werden unter den unter folgenden INCI-Bezeichnungen bekannten polymeren Verdickungsmitteln: Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis

Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.
Besonders bevorzugt wird der polymere Verdicker ausgewählt unter polymeren, anionischen, amphiphilen Verdickern, besonders bevorzugt unter solchen mit den INCI-Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates / Palmeth-20 Acrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylates / Steareth-20 Methacrylate Crosspolymer.

Die polymeren Verdickungsmittel sind bevorzugt in einer Menge von 0,5 bis 20 Gew.-% insbesondere von 0,5 bis 10 Gew.-% in dem erfindungsgemäßen Mittel in Cremeform oder Gelform enthalten.
Dabei handelt es sich erfindungsgemäß bevorzugt um ein transparentes Gel.

Erfindungsgemäß ganz besonders bevorzugte kosmetische Mittel in Gelform gehorchen mindestens einer der folgenden Ausführungsformen A-2) bis I-2):

A-2):
Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel, mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 1 bis 20 Gew.-% aufweist.

B-2):
Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel,

mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 8,0 bis 12,0 Gew.-% aufweist.

C-2):
Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel, mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 9,5 bis 10,5 Gew.-% oder 4 bis 6 Gew.-% aufweist.

D-2):
Kosmetisches Mittel zur temporären Umformung keratinischer Fasern, insbesondere menschlicher Haare, enthaltend in einem in Form eines Gels vorliegenden, kosmetischen Träger, umfassend mindestens ein polymeres Verdickungsmittel,

(a) mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 1 bis 20 Gew.-% aufweist und
(b) mindestens ein zusätzliches filmbildendes und/oder festigendes Polymer.

E-2):
Kosmetisches Mittel zur temporären Umformung keratinischer Fasern, insbesondere menschlicher Haare, enthaltend in einem in Form eines Gels vorliegenden, kosmetischen Träger, umfassend mindestens ein polymeres Verdickungsmittel,

(a) mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 4 bis 12 Gew.-% aufweist und
(b) mindestens ein zusätzliches filmbildendes und/oder festigendes Polymer.

F-2):
Kosmetisches Mittel zur temporären Umformung keratinischer Fasern, insbesondere menschlicher Haare, enthaltend in einem in Form eines dispersen Systems vorliegenden, kosmetischen Träger

- mindestens eine mit Propylenoxid modifizierte Stärke mit einem mittleren Molekulargewicht (Gewichtsmittel) von 700 bis 1000 kDa, welche - bezogen auf das Gewicht besagter Stärke - einen Propylenoxidgehalt von 9,5 bis 10,5 Gew.-% oder 4 bis 6 Gew.-% aufweist und
- mindestens ein zusätzliches filmbildendes und/oder festigendes Polymer.

G-2):
Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel, mindestens eine mit Propylenoxid modifizierte Stärke, welche ein mittleres Molekulargewicht (Gewichtsmittel) von 700 bis 1000 kDa sowie - bezogen auf das Gewicht besagter Stärke - einen Propylenoxidgehalt von 1 bis 20 Gew.-% aufweist.

H-2):
Kosmetisches Mittel zur temporären Umformung keratinischer Fasern, insbesondere menschlicher Haare, Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel,

mindestens eine mit Propylenoxid modifizierte Stärke welche ein mittleres Molekulargewicht (Gewichtsmittel) von 700 bis 1000 kDa sowie - bezogen auf das Gewicht besagter Stärke - einen Propylenoxidgehalt von 4 bis 12 Gew.-% aufweist.
I-2):
Kosmetisches Mittel zur temporären Umformung keratinischer Fasern, insbesondere menschlicher Haare, Kosmetisches Mittel zur temporären Umformung keratinischer Fasern, insbesondere menschlicher Haare, enthaltend in einem in Form eines Gels vorliegenden, kosmetischen Träger, umfassend

- mindestens ein polymeres Verdickungsmittel,

mindestens eine mit Propylenoxid modifizierte Stärke welche ein mittleres Molekulargewicht (Gewichtsmittel) von 700 bis 1000 kDa sowie - bezogen auf das Gewicht besagter Stärke - einen Propylenoxidgehalt von 9,5 bis 10,5 Gew.-% oder 4 bis 6 Gew.-% aufweist.

Im Rahmen der zuvor genannten Ausführungsformen gelten selbstverständlich *mutatis mutandis* die jeweiligen als bevorzugt gekennzeichneten Merkmale des erfindungsgemäßen Mittels, wie insbesondere die Einsatzmengen.
Weiterhin sind insbesondere auch im Rahmen der Ausführungsformen A-2) bis I-2) als polymeres Verdickungsmittel insbesondere anionische, amphiphile, polymere Verdickungsmittel bevorzugt.
Als insbesondere bevorzugtes zusätzliches filmbildendes und/oder festigendes Polymer im Rahmen der Ausführungsformen A-2) bis I-2) gilt mindestens ein Copolymer gemäß Anspruch 14.
Wenn die erfindungsgemäßen Mittel als Schaum vorliegen sollen, werden sie aus einer zum Verschäumen geeigneten Abgabevorrichtung ausgebracht, die entweder ein zusätzlich mit einem Treibmittel befüllter Druckgasbehälter ("Aerosolbehälter") oder ein Nichtaerosolbehälter ist. In diesem Falle liegt das disperse System des kosmetischen Trägers als Schaum vor.
Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.
Insbesondere bevorzugt liegen die erfindungsgemäßen Mittel als Aerosolhaarschaum vor. Das erfindungsgemäße Mittel enthält daher bevorzugt zusätzlich mindestens ein Treibmittel.
Erfindungsgemäße Mittel, die in Form eines Aerosolprodukts vorliegen, lassen sich in üblicher Art und Weise herstellen. In der Regel werden alle Bestandteile des erfindungsgemäßen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.
Zur Erzeugung eines erfindungsgemäß bevorzugten Aerosolschaums erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.
Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$, Dimethylether, $CO_2$, Luft, Alkanen mit 3 oder 4 Kohlenstoffatomen (wie Propan, n-Butan, iso-Butan) sowie Mischungen daraus.
Über das Mengenverhältnis von Treibmittel zu den übrigen Bestandteilen der Zubereitungen lassen sich bei gegebener Sprühvorrichtung die Größen der Schaumblasen und die jeweilige Größenverteilung einstellen.
Bei Verwendung herkömmlicher Aerosolbehälter enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt.
Der Einsatz der zuvor genannten zusätzlichen bevorzugten Inhaltsstoffe und der als bevorzugt gekennzeichneten Einsatzmengen bzw. Einsatzmengenverhältnisse (*vide supra*) sind natürlich auch im Rahmen dieser Ausführungsform(en) bevorzugt.
Ein zweiter Gegenstand der Erfindung ist die Verwendung eines kosmetischen Mittels des ersten Erfindungsgegenstandes zur temporären Umformung und/oder Formfixierung keratinischer Fasern, insbesondere menschlicher Haare.

Ein dritter Gegenstand der Erfindung ist ein Verfahren zur temporären Umformung keratinischer Fasern, insbesondere menschlicher Haare, dadurch gekennzeichnet, dass ein kosmetisches Mittel des ersten Erfindungsgegenstandes auf die keratinischen Fasern aufgebracht wird.
Es hat sich als bevorzugt herausgestellt, wenn die keratinischen Fasern nach der Einwirkung der kosmetischen Mittel des ersten Erfindungsgegenstandes nicht gespült und auf der Faser belassen werden.
Die nachfolgenden Beispiele sollen den Gegenstand der vorliegenden Erfindung erläutern ohne ihn in irgendeiner Weise zu beschränken.

**Beispiele**

Es wurden folgende Handelsprodukte verwendet:

| | |
|---|---|
| Abil Quat 3272 | 3-(3-((3-Cocoamidopropyl)dimethylammonio)-2-hydroxypropyl)propylgruppen terminiertes Polysiloxan (50 Gew.-& Aktivsubstanz in 1,2-Propylenglykol; INCI-Bezeichnung: Quaternium-80) (Goldschmidt) |
| Advantage® LC-A | Vinylcaprolactam Vinylpyrrolidon Dimethylaminoethylmethacrylat Copolymer (Ca.35-39% Festkörper in Ethanol; INCI-Bezeichnung: Vinyl Caprolactam/VP/Dimethylaminoethyl Methacrylate Copolymer, Alcohol) (ISP) |
| Aculyn® 28 | Copolymer aus (Meth)acrylsäure, (Meth)acrylsäureester und Beheneth-25-Methacrylsäureester (19-21 Gew.-% Festkörper in Wasser; INCI-Bezeichnung: Acrylates/Beheneth-25 Methacrylate Copolymer) (Rohm und Haas) |
| Amaze | nichtionische, modifizierte Maisstärke (INCI-Bezeichnung: Corn Starch Modified) (National Starci) |
| Antaron® WP-660 | Copolymer aus Vinylpyrrolidon und 1-Triaconten (INCI-Bezeichnung: Tricontanyl PVP) (ISP) |
| Celquat® L 200 | quaterniertes Cellulose-Derivat (INCI-Bezeichnung: Polyquaternium-4) (National Starch) |
| Crodafos® CES | INCI-Bezeichnung: Cetearyl Alcohol, Dicetyl Phosphate, Ceteth-10 Phosphate (Croda) |
| Dekafald | 1,3-Dihydroxymethyl-5,5-dimethylhydantoin (ca. 54-56 Gew.-% Aktivsubstanz in Wasser; INCI-Bezeichnung: DMDM Hydantoin) (Jan Dekker) |
| Dow Corning® 556 | Polyphenylmethylsiloxan (INCI-Bezeichnung: Phenyl Trimethicone) (Dow Corning) |
| Edenor L2SM | Mischung aus Palmitinsäure und Stearinsäure (INCI-Bezeichnung: Palmitic Acid, Stearic Acid) (Cognis) |
| Euxyl® K 320 | Mischung aus Phenoxyethanol, Methylparaben, Ethylparaben, Propylenglykol (Schülke & Mayr) |
| Dow Corning® 939 | ca. 32-36% Festkörper; INCI-Bezeichnung: Amodimethicone, Trideceth-12, Cetrimonium Chloride (Dow Corning) |
| Dow Corning® 959 | 52-37% Festkörper; INCI-Bezeichnung: Amodimethicone, Trideceth-12, Cetrimonium Chloride (Dow Corning) |
| Gafquat® 755N PW | Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP) |
| Genamin® CTAC | Trimethylhexadecylammoniumchlorid (ca. 28-30% Aktivsubstanz in Wasser; INCI-Bezeichnung: Cetrimonium Chloride) (Clariant) |

| | |
|---|---|
| Luviskol® VA 64 W | Copolymer aus Vinylpyrrolidon und Vinylacetat (60:40) (48-52% Aktivsubstanz in Wasser, INCI-Bezeichnung: VP/VA Copolymer) (BASF) |
| Luviskol® K85 | Polyvinylpyrrolidon (ca. 20% Festkörper in Wasser; INCI-Bezeichnung: PVP) (BASF) |
| Luviskol® K90 | Polyvinylpyrrolidon (ca. 20% Festkörper in Wasser; INCI-Bezeichnung: PVP) (BASF) |
| Luviskol® VA 73 W | Vinylpyrrolidon Vinylacetat Copolymer (70:30) (ca. 48-52% Festkörper in Wasser; INCI-Bezeichnung: VP/VA Copolymer) (BASF) |
| Neolone® PE | 2-Methyl-2H-isothiazolin-3-one (ca. 1,55% in 2-Phenoxyethanol; INCI-Bezeichnung: Phenoxyethanol, Methylisothiazolinone) (Rohm & Haas) |
| Pemulen® TR-1 | verzweigtes Acrylsäurepolymer (INCI-Bezeichnung: Acrylates/C10-30 Alkyl Acrylate Crosspolymer) (Lubrizol) |
| Silsoft Q | 31 Gew.-% in Wasser (INCI-Bezeichnung: Aqua (Water), Silicone Quaternium-18, Trideceth-6, Trideceth-12) (Momentive) |
| Styleze® CC 10 | Copolymer aus N-Vinylpyrrolidon und N,N-Dimethylaminopropylmethacrylamid (ca. 9 bis 11 % Aktivsubstanz, INCI-Bezeichnung:VP/DMAPA Acrylates Copolymer) (ISP) |
| Uvinul® P 25 | 4-Aminobenzoesäureethylester + 25 Mol Ethylenoxid (INCI-Bezeichnung: PEG-25 PABA) (BASF SE) |
| Tylose H 100000 YP2 | Hydroxyethylcellulose (90 Gew.-% Aktivsubstanz; INCI-Bezeichnung: Hydroxyethylcellulose) (SE Tylose) |
| Xiameter PMX 200 Sil Fluid | Polydimethylsiloxan (INCI-Bezeichnung: Dimethicone, Viskosität: 50 |
| 50 CS | cSt) (Xiameter) |

1.1 **Styling Cremes**

| Rohstoff | V1 | E1 | E2 | E3 |
|---|---|---|---|---|
| Dinatrium EDTA | 0,05 | 0,05 | - | - |
| 1,2-Propandiol | 1,50 | 1,50 | - | - |
| mit Propylenoxid modifizierte Stärke [1] | - | 30,00 | 3,00 | 20,00 |
| Methylparaben | 0,13 | 0,13 | 0,10 | - |
| Propylparaben | 0,03 | 0,03 | - | - |
| Tylose H 100000 YP2 | 0,30 | 0,30 | - | - |
| Amaze | 0,50 | 0,50 | - | - |
| Pemulen TR-1 | - | - | 0,30 | 0,20 |
| Aculyn 88 | - | - | 6,00 | - |
| Weichceresin FL 400 | 2,00 | 2,00 | - | 6,00 |
| Crodafos CES | 1,50 | 1,50 | - | 1,00 |
| Estol 3752 | 0,30 | 0,30 | - | - |
| Cetylalkohol | 0,75 | 0,75 | - | 2,00 |
| Diisopropyladipat | - | - | 0,20 | - |
| Benzoesäure-($C_{12}$ bis $C_{15}$)-Alkylester | - | - | - | 5,00 |

(fortgesetzt)

| Rohstoff | V1 | E1 | E2 | E3 |
|---|---|---|---|---|
| Paraffinum Liquidum | - | - | - | 1,00 |
| Bienenwachs | - | - | - | 5,00 |
| Xiameter PMX 200 Sil Fluid 50 CS | - | - | 0,30 | 0,50 |
| Silsoft Q | - | - | 0,50 | - |
| Dow Corning 556 | - | - | 1,00 | - |
| Doe Corning 959 | - | - | 0,50 | - |
| Abil Quat 3272 | - | - | 1,00 | - |
| Cetyltrimethylammoniumchlorid | - | - | 0,30 | - |
| Antaron WP-660 | 1,50 | 1,50 | - | - |
| Stearylalkohol | 0,75 | 0,75 | - | - |
| Steareth-21 | 0,75 | 0,75 | - | 1,10 |
| Edenor L2SM | - | - | - | 7,50 |
| Steareth-2 | - | - | - | 1,50 |
| Oleth-20 | - | - | - | 1,00 |
| PPG-5-Ceteth-20 | - | - | - | 1,00 |
| ADVANTAGE LC-A | 10,00 | - | - | - |
| Luviskol VA 73 W | 10,00 | - | - | - |
| Luviskol K 85 | - | 10,00 | - | - |
| Luviskol K 90 | 20,00 | - | - | - |
| Styleze CC-10 | 10,00 | 10,00 | - | - |
| Dekafald | 0,30 | - | - | - |
| 2-Phenoxyethanol | - | 0,50 | - | 0,40 |
| Glyzerin | - | - | 1,50 | - |
| D-Panthenol | - | - | 0,20 | - |
| Neolone PE | - | - | 0,30 | 0,50 |
| 2-Amino-2-methylpropanol | - | - | 0,50 | - |
| Milchsäure | - | 2,00 | - | - |
| Parfum | 0,70 | 0,70 | 0,30 | 0,90 |
| Wasser | ad 100 | ad 100 | ad 100 | ad 100 |
| [1] Tapiokastärke mit 10 Gew.-% Propylenoxid modifiziert, mittleres Molekulargewicht 800 kDa, Viskosität einer 43 Gew.-%igen Lösung in Wasser 64000 mPas | | | | |

1.2 **Styling Gele**

| Rohstoff | E4 | E5 | E6 | E7 |
|---|---|---|---|---|
| Dinatrium EDTA | - | - | 0,05 | - |
| 1,2-Propandiol | 6,00 | - | - | - |
| mit Propylenoxid modifizierte Stärke [1] | 9,50 | 12,00 | 12,00 | 2,00 |
| Methylparaben | 0,10 | - | - | |

(fortgesetzt)

| Rohstoff | E4 | E5 | E6 | E7 |
|---|---|---|---|---|
| Propylparaben | - | - | - | |
| Aculyn 28 | 3,00 | - | - | |
| Aculyn 88 | - | - | - | 2,30 |
| Synthalen W 2000 | - | - | - | 1,80 |
| Polyqel W 30 | - | 3,00 | - | - |
| Structure 2001 | - | - | 5,00 | - |
| PEG-40 Hydroqenated Castor Oil | 0,40 | - | - | 0,20 |
| PPG-5-Ceteth-20 | - | - | 0,50 | - |
| Euxyl K320 | - | - | - | 1,00 |
| Luviskol K 90 | - | - | 12,00 | - |
| Luviskol K 85 | - | 5,00 | - | - |
| Polyethylenglykol 1500 | 2,00 | - | - | - |
| Dekafald | - | - | - | - |
| 2-Phenoxyethanol | - | - | - | - |
| Glyzerin | 10,00 | 2,75 | 2,00 | - |
| D-Panthenol | 0,15 | - | 0,20 | 0,20 |
| Neolone PE | 0,30 | 0,60 | 0,60 | |
| Triethanolamin | - | - | - | 0,80 |
| Milchsäure | - | 0,10 | 0,30 | - |
| Uvinul P25 | - | - | - | 0,05 |
| Parfum | 0,15 | 0,20 | 0,20 | 0,20 |
| Wasser | ad 100 | ad 100 | ad 100 | ad 100 |
| [1] Tapiokastärke mit 10 Gew.-% Propylenoxid modifiziert; mittleres Molekulargewicht 800 kDa, Viskosität einer 43 Gew.-%igen Lösung in Wasser 64000 mPas | | | | |

1.3 **Styling Schäume (Aerosol)**

| Rohstoff | V2 | E7 |
|---|---|---|
| Celquat L 200 | 0,30 | 0,30 |
| Luviskol VA 64 W | 2,00 | - |
| mit Propylenoxid modifizierte Stärke [1] | - | 2,00 |
| Gafquat 755N PW | 7,40 | 7,40 |
| D-Panthenol | 0,20 | 0,20 |
| Glyzerin | 0,15 | 0,15 |
| Dow Corning 939 | 0,20 | 0,20 |
| Genamin CTAC | 1,00 | 1,00 |
| PEG 40 Hydrogenated Castor Oil | 0,30 | 0,30 |
| Parfum | 0,15 | 0,20 |

(fortgesetzt)

| Rohstoff | V2 | E7 |
|---|---|---|
| Wasser | ad 100 | ad 100 |
| [1] Tapiokastärke mit 10 Gew.-% Propylenoxid modifiziert; mittleres Molekulargewicht 800 kDa, Viskosität einer 43 Gew.-%igen Lösung in Wasser 64000 mPas | | |

Die Rezepturen V2 und E7 wurden jeweils in ein Aerosolbehältnis gefüllt, das folgende technische Parameter erfüllt: Aluminium Vorratsbehältnis mit Ventil Produkt 522983 PV10697 der Firma Pecision (Deutsche Präzisions-Ventil GmbH).
Das Aerosolbehältnis wurde mit einem Treibgasgemisch aus Propan/Butan (47 Gew.-% Propan, 50 Gew.-% Butan, 3 Gew.-% Isobutan) befüllt, sodass sich ein Gewichtsverhältnis von Rezeptur zu Treibgas von 92 zu 8 ergab.

2.0 **Wirkungsnachweis**

Es wurden exemplarisch aus den oben angeführten Zusammensetzungen die Mittel E1, E7, V1 und V2 miteinander verglichen. Folgende Ergebnisse wurden erhalten:

a) **Haarschäume**

a1) Halt, Elastizität und Flexibilität (Omega Loop-Methode)

Tabelle 2: Halt, Elastizität, Flexibilität

| Zusammensetzung | F max (N) | Elastizität | Flexibilität |
|---|---|---|---|
| E7 | 2,2 | 60 | 75 |
| V2 | 1,9 | 50 | 80 |

Je größer die F max-Werte, umso besser ist der Frisurenhalt. Die Werte der Tabelle 2 belegen, dass die erfindungsgemäßen Propylenoxid modifizierten Stärkederivate einen zum gängigen
Filmbildner PVP/VA vergleichbaren - bei niederem Molekulargewicht der Propylenoxid modifizierten Stärkederivate sogar besseren - Frisurenhalt ergeben.
Je größer die Werte der Elastizität bzw. der Flexibilität, umso besser ist die Elastizität bzw. die Flexibilität der Frisur. Die mit den erfindungsgemäßen Mitteln behandelten Haarsträhnen wiesen einen flexibleren und elastischeren Halt auf, als die Strähnen die mit PVP/VA-Copolymer behandelt wurden.

a2) High Humidity Curl Retention (HHCR)

Tabelle 3: Lockenerhalt bei hoher Luftfeuchtigkeit (HHCR)

| Zusammensetzung | HHCR |
|---|---|
| E7 | 50 |
| V2 | 20 |

Die mit dem erfindungsgemäßen Mittel behandelten Haarsträhnen wiesen einen gegen Luftfeuchtigkeit beständigeren Halt der Frisur auf.

b) **Haargele**

b1) Halt, Elastizität, Flexibilität

Tabelle 4: Halt, Elastizität, Flexibilität

| Zusammensetzung | F max (N) | Elastizität | Flexibilität |
|---|---|---|---|
| E1 | 4,1 | 66 | 88 |
| V1 | 3,3 | 55 | 85 |

Je größer die F max-Werte, umso besser ist der Frisurenhalt. Die Werte der Tabelle 4 belegen, dass die erfindungsgemäßen Propylenoxid modifizierten Stärkederivate einen zum gängigen Filmbildner PVP/VA vergleichbaren - bei niederem Molekulargewicht der Propylenoxid modifizierten Stärkederivate sogar besseren - Frisurenhalt ergeben.

Je größer die Werte der Elastizität bzw. der Flexibilität, umso besser ist die Elastizität bzw. die Flexibilität der Frisur. Die mit den erfindungsgemäßen Mitteln behandelten Haarsträhnen wiesen einen flexibleren und elastischeren Halt auf, als die Strähnen die mit PVP/VA-Copolymer behandelt wurden.

3.0 **Durchführung der Omega Loop-Messung**

Der Halt der Verformung, auch als Frisurenhalt bezeichnet, sowie Flexibilität und Elastizität werden im Sinne der vorliegenden Erfindung nach der Omega-Loop Methode bestimmt.

Dazu wird eine trockene Haarsträhne (Euro-Naturhaar der Firma Kerling, Klebetresse dicht, einseitig geklebt, Gesamtlänge 160 mm, freie Länge 150 mm, Breite 10 mm, Gewicht 1,0 ± 0,1 g) für 30 Sekunden bis zum unteren Rand der Abklebung in die zu untersuchende Polymerlösung getaucht. Anschließend wird die überschüssige Lösung zwischen Daumen und Zeigefinger abgestrichen, so dass 0,5 ± 0,02 g der Lösung auf dem Haar verbleiben. Die mit der zu untersuchenden Lösung gesättigte Haarsträhne wird um einen Teflon-Zylinder mit einem Durchmesser von 36 mm gewickelt und die überstehenden Enden werden mit einem Clip fixiert. Die präparierten Strähnen werden anschließend über Nacht bei 25°C und 50% relativer Luftfeuchte oder bei 25°C und 75% relativer Luftfeuchte im Klimaschrank getrocknet und konditioniert.

Die konditionierte Strähne wird vorsichtig von dem Teflon-Zylinder entfernt. Der entstandene Ω-Loop, eine ringförmige Struktur des in seiner Form durch den ausgebildeten Polymerfilm stabilisierten Haars, wird in den an der Messdose befestigten Greifer eingespannt und bis dicht über die Bodenplatte eines Universalprüfgeräts AMETEK LF Plus der Firma AMETEK Precision Instuments Europe GmbH, Produktgruppe Lloyd abgesenkt. Die gesamte Messung erfolgt im Klimaschrank unter konstanten klimatischen Bedingungen bei 25°C und 50% relativer Luftfeuchte oder bei 25°C und 75% relativer Luftfeuchte.

Um standardisierte Ausgangsbedingungen zu schaffen, startet die Messung mit dem Anfahren einer Vorlast von 0,07 N mit einer Geschwindigkeit von 30 mm $min^{-1}$. Anschließend wird der Ω-Loop mit einer Geschwindigkeit von 60 mm $min^{-1}$ um 8 mm gestaucht, wobei die dazu nötige Kraft gemessen wird. Nachdem die charakteristische Kraft $F_1$ bei der maximalen Deformation von 8 mm aufgezeichnet wurde, wird die Strähne mit 60 mm $min^{-1}$ soweit entlastet, dass sie 10 mm von der Bodenplatte abhebt. Von hier aus beginnt der nächste Zyklus, indem erneut die Vorlast von 0,07 N angefahren und die Strähne anschließend um 8 mm gestaucht wird, hierbei gelten die gleichen Geschwindigkeiten wie oben beschrieben. Die Messung eines Ω-Loops umfasst insgesamt 10 Zyklen.

Mit dieser Messmethode lassen sich vier charakteristische Parameter zur Beschreibung der mechanischen Eigenschaften von filmbildenden Polymeren bestimmen. Halt, Flexibilität, Plastizität und Elastizität lassen sich nach folgenden Formeln aus den gemessenen Kräften berechnen:

$$\textit{Halt} = F_1\ [N]$$

($F_1$ entspricht der Maximalkraft der Messung)

$$\textit{Flexibilität} = \frac{F_{10}}{F_1}$$

(gibt das Verhältnis der Maximalkräfte des zehnten zum ersten Zyklus an)

$$Elastizität = \frac{\frac{F_{10}(2mm) - F_{10}(1,5mm)}{0,5}}{\frac{F_{1}(2mm) - F_{1}(1,5mm)}{0,5}} = \frac{E_{10}}{E_{1}}$$

(zur Berechnung der Elastizität werden aus dem ersten und zehnten Zyklus jeweils die Kräfte zur Verformung um 1,5 mm und 2 mm erfasst und miteinander ins Verhältnis gesetzt).

4.0 **Durchführung der High Humidity Curl Retention-Messung**

Es wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18g der Zusammensetzungen wurden auf jeweils eine Haarsträhne appliziert und einmassiert. Die Strähne wurde sodann auf einen Wickler gewickelt (Fripac-medis, Ø 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 24 h gelagert und danach die Längen der Locken erneut vermessen ($L_t$).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{max} - L_t}{L_{max} - L_0}$$

## Patentansprüche

1. Kosmetisches Mittel zur temporären Umformung keratinischer Fasern, insbesondere menschlicher Haare, enthaltend in einem in Form eines dispersen Systems vorliegenden, kosmetischen Träger mindestens eine mit Propylenoxid modifizierte Stärke, welche ein mittleres Molekulargewicht (Gewichtsmittel) von 700 bis 1000 kDa besitzt, wobei es die mit Propylenoxid modifizierte Stärke in einer Menge von 2 Gew.-% bis 10 Gew.-%, bezogen auf das Gewicht des Mittels, enthält.

2. Kosmetisches Mittel nach Anspruch 1, **dadurch gekennzeichnet, dass** die mit Propylenoxid modifizierte Stärke einen Propylenoxidgehalt von 1 bis 20 Gew.-%, insbesondere von 5 bis 15 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder 4 bis 6 Gew.-%, - jeweils bezogen auf das Gewicht der besagten Stärke - aufweist.

3. Kosmetisches Mittel nach einem der Ansprüche 1 oder 2, **dadurch gekennzeichnet, dass** das mit Propylenoxid modifizierte Polysaccharid in einer 43 Gew.-%-igen wässrigen Lösung eine Viskosität im Bereich von 150 bis 1500000 mPa·s, bevorzugt von 10000 bis 200000 mPa·s, besonders bevorzugt von 25000 bis 180000 mPa·s (jeweils Brookfield Viskosimeter, Spindel #7 bei 20°C und 20 U/min) aufweist.

4. Kosmetisches Mittel nach einem der Ansprüche 1 bis 3, **dadurch gekennzeichnet, dass** die mit Propylenoxid modifizierte Stärke einen Amylosegehalt von kleiner 25 Gew.-%, insbesondere von kleiner 20 Gew.-%, - jeweils bezogen auf das Gewicht der besagten Stärke - besitzt.

5. Kosmetisches Mittel nach einem der Ansprüche 1 bis 4, **dadurch gekennzeichnet, dass** es als mit Propylenoxid modifizierte Stärke mindestens eine unvernetzte, mit Propylenoxid modifizierte Stärke enthalten.

6. Kosmetisches Mittel nach einem der Ansprüche 1 bis 5, **dadurch gekennzeichnet, dass** es als Creme oder Gel vorliegt.

7. Kosmetisches Mittel nach Anspruch 6, **dadurch gekennzeichnet, dass** es zusätzlich mindestens einen polymeres Verdickungsmittel enthält.

8. Kosmetisches Mittel nach Anspruch 7, **dadurch gekennzeichnet, dass** der polymere Verdicker ausgewählt wird unter polymeren, anionischen, amphiphilen Verdickern (bevorzugt unter solchen mit den INCI-Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates / Palmeth-20 Acrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylates / Steareth-20 Methacrylate Crosspolymer).

9. Kosmetisches Mittel nach einem der Ansprüche 1 bis 5, **dadurch gekennzeichnet, dass** es als Schaum vorliegt und aus einer zum Verschäumen geeigneten Abgabevorrichtung ausgebracht wird, die entweder ein zusätzlich mit einem Treibmittel befüllter Druckgasbehälter ("Aerosolbehälter") oder ein Nichtaerosolbehälter ist.

10. Kosmetisches Mittel nach einem der Ansprüche 1 bis 9, **dadurch gekennzeichnet, dass** es die mit Propylenoxid modifizierte Stärke in einer Menge von 2 Gew.-% bis 8 Gew.-%, ganz besonders bevorzugt von 3 Gew.-% bis 6 Gew.-%, jeweils bezogen auf das Gewicht des Mittels, enthält.

11. Kosmetisches Mittel nach einem der Ansprüche 1 bis 9, **dadurch gekennzeichnet, dass** es zusätzlich mindestens ein filmbildendes und/oder festigendes Polymer enthält.

12. Kosmetisches Mittel nach Anspruch 11, **dadurch gekennzeichnet, dass** es als zusätzliches filmbildendes und/oder festigendes Polymer mindestens ein Copolymer enthält, das gebildet wird aus

- mindestens einem Monomer (Mo1) ausgewählt aus Acrylsäure, Methacrylsäure, Acrylsäurealkylestern und Methacrylsäurealkylestern, und
- mindestens einem amphoteren Monomer (Mo2) ausgewählt aus (Meth)acryloylalkyl-aminoxiden der Formel (Mo2)

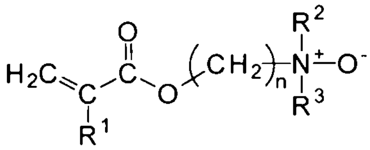

(Mo2)

worin

- $R^1$ für H oder $CH_3$,
- $R^2$ und $R^3$ jeweils unabhängig voneinander für gegebenenfalls verzweigtes $C_{1-10}$-Alkyl und
- n für eine ganze Zahl von 1 bis 20 steht.

13. Verwendung eines kosmetischen Mittels nach einem der Ansprüche 1 bis 8 zur temporären Umformung und/oder Formfixierung keratinischer Fasern, insbesondere menschlicher Haare.

**Claims**

1. A cosmetic agent for temporarily shaping keratin fibres, in particular human hair, containing, in a cosmetic carrier in the form of a disperse system, at least one propylene-oxide-modified starch that has an average molecular mass (weight average) of from 700 to 1000 kDa, wherein said agent contains the propylene-oxide-modified starch in an amount of from 2 wt.% to 10 wt.%, based on the weight of the agent.

2. The cosmetic agent according to claim 1, **characterized in that** the propylene-oxide-modified starch has a propylene oxide content of from 1 to 20 wt.%, in particular of from 5 to 15 wt.%, particularly preferably a propylene oxide content of from 4 to 12 wt.%, very particularly preferably a propylene oxide content of from 9.5 to 10.5 wt.% or from 4 to 6 wt.%, based in each case on the weight of said starch.

3. The cosmetic agent according to one of claims 1 or 2, **characterized in that** the propylene-oxide-modified polysaccharide has, in a 43 wt.% aqueous solution, a viscosity in the range of from 150 to 1500000 mPas, preferably of from 10000 to 200000 mPas, particularly preferably of from 25000 to 180000 mPas (in each case Brookfield viscometer, spindle #7 at 20 °C and 20 rpm).

4. The cosmetic agent according to one of claims 1 to 3, **characterized in that** the propylene-oxide-modified starch has an amylose content of less than 25 wt.%, in particular of less than 20 wt.%, based in each case on the weight of said starch.

5. The cosmetic agent according to one of claims 1 to 4, **characterized in that** it contains at least one non-crosslinked propylene-oxide-modified starch as the propylene-oxide-modified starch.

6. The cosmetic agent according to one of claims 1 to 5, **characterized in that** it is a cream or a gel.

7. The cosmetic agent according to claim 6, **characterized in that** it additionally contains at least one polymeric thickener.

8. The cosmetic agent according to claim 7, **characterized in that** the polymeric thickener is selected from polymeric, anionic, amphiphilic thickeners (preferably from those having the INCI names Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates / Palmeth-20 Acrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylates / Steareth-20 Methacrylate Crosspolymer).

9. The cosmetic agent according to one of claims 1 to 5, **characterized in that** it is in the form of a foam and extracted from a dispensing device capable of foaming, which is either a compressed-gas container filled with a propellant ("aerosol container") or a non-aerosol container.

10. The cosmetic agent according to one of claims 1 to 9, **characterized in that** it contains the propylene-oxide-modified starch in an amount of from 2 wt.% to 8 wt.%, very particularly preferably of from 3 wt.% to 6 wt.%, based in each case on the weight of the agent.

11. The cosmetic agent according to one of claims 1 to 9, **characterized in that** it additionally contains at least one film-forming and/or stabilizing polymer.

12. The cosmetic agent according to claim 11, **characterized in that** it contains, as the additional film-forming and/or stabilizing polymer, at least one copolymer that is formed from

- at least one monomer (Mo1) selected from acrylic acid, methacrylic acid, acrylic acid alkyl esters and methacrylic acid alkyl esters, and
- at least one amphoteric monomer (Mo2) selected from (meth)acryloyl alkyl amine oxides of the formula (Mo2)

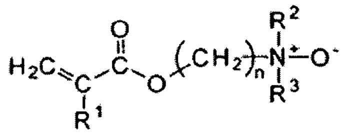

(Mo2)

in which

- $R^1$ represents H or $CH_3$,
- $R^2$ and $R^3$ each independently represent optionally branched $C_{1-10}$-alkyl, and
- n represents a whole number from 1 to 20.

**13.** Use of a cosmetic agent according to one of claims 1 to 8 for temporarily shaping and/or setting the shape of keratin fibres, in particular human hair.

**Revendications**

**1.** Produit cosmétique pour la déformation temporaire de fibres de kératine, en particulier de cheveux humains, contenant dans un vecteur cosmétique présent sous forme d'un système dispersé au moins un amidon modifié par de l'oxyde de propylène, possédant un poids moléculaire moyen (en poids) entre 700 et 1 000 kDa, l'amidon modifié par de l'oxyde de propylène étant contenu à hauteur de 2 à 10 % en poids rapporté au poids du produit.

**2.** Produit cosmétique selon la revendication 1, **caractérisé en ce que** l'amidon modifié par de l'oxyde de propylène présente une teneur en oxyde de propylène entre 1 et 20 % en poids, en particulier entre 5 et 15 % en poids, particulièrement préférentiellement entre 4 et 12 % en poids, tout particulièrement préférentiellement entre 9,5 et 10,5 % en poids ou entre 4 et 6 % en poids, rapporté au poids dudit amidon.

**3.** Produit cosmétique selon la revendication 1 ou 2, **caractérisé en ce que** le polysaccharide modifié par de l'oxyde de propylène présente dans une solution aqueuse à 43 % une viscosité entre 150 et 1 500 000 mPa.s, préférentiellement entre 10 000 et 200 000 mPa.s, particulièrement préférentiellement entre 25 000 et 180 000 mPa.s (viscosimètre Brookfield, rotor n°7, à 20°C et 20 tr/min).

**4.** Produit cosmétique selon une des revendications 1 à 3, **caractérisé en ce que** l'amidon modifié par de l'oxyde de propylène possède une teneur en amylose inférieure à 25 % en poids, en particulier inférieure à 20 % en poids rapporté au poids dudit amidon.

**5.** Produit cosmétique selon une des revendications 1 à 4, **caractérisé en ce qu'**il contient comme amidon modifié par de l'oxyde de propylène au moins un amidon non-réticulé modifié par de l'oxyde de propylène.

**6.** Produit cosmétique selon une des revendications 1 à 5, **caractérisé en ce qu'**il constitue une crème ou un gel.

**7.** Produit cosmétique selon la revendication 6, **caractérisé en ce qu'**il contient en plus au moins un épaississant polymère.

**8.** Produit cosmétique selon la revendication 7, **caractérisé en ce que** l'épaississant polymère est choisi parmi des épaississants polymères, anioniques, amphiphiles (préférentiellement parmi ceux ayant comme désignation INCI : Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-20 Acrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylates/Steareth-20 Methacrylate Crosspolymer).

**9.** Produit cosmétique selon une des revendications 1 à 5, **caractérisé en ce qu'**il constitue une mousse et **en ce qu'**on l'applique à partir d'un distributeur adéquat pour le faire mousser, qui est soit un récipient de gaz rempli en plus avec un vecteur ("flacon aérosol"), soit un récipient qui ne produit pas d'aérosol.

**10.** Produit cosmétique selon une des revendications 1 à 9, **caractérisé en ce qu'**il contient l'amidon modifié par de l'oxyde de propylène à hauteur de 2 à 8 % en poids, tout particulièrement préférentiellement de 3 à 6 % en poids, rapporté au poids du produit.

**11.** Produit cosmétique selon une des revendications 1 à 9, **caractérisé en ce qu'**il contient en plus au moins un polymère filmogène et/ou durcisseur.

**12.** Produit cosmétique selon la revendication 11, **caractérisé en ce qu'**il contient comme polymère supplémentaire

filmogène et/ou durcisseur au moins un copolymère formé de :

- au moins un monomère (Mo1) choisi parmi l'acide méthacrylique, les acrylates d'alkyle et les méthacrylates d'alkyle, et
- au moins un monomère amphotère (Mo2) choisi parmi les oxydes de (méth)acrylates d'alkylamine de formule (Mo2) :

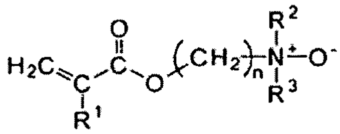

(Mo2)

où

- $R^1$ représente H ou $CH_3$
- $R^2$ et $R^3$ représentent respectivement, indépendamment l'un de l'autre, un alkyle en $C_{1-10}$ éventuellement ramifié, et
- n représente un nombre entier entre 1 et 20.

**13.** Utilisation d'un produit cosmétique selon une des revendications 1 à 8 pour la déformation temporaire et/ou la fixation de forme de fibres de kératine, en particulier de cheveux humains.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS19738866 A